# EUROPÄISCHE PATENTSCHRIFT

(11) **EP 1 853 386 B1**
(45) Veröffentlichungstag und Bekanntmachung des Hinweises auf die Patenterteilung: **11.09.2019**
(21) Anmeldenummer: 06707386.6
(22) Anmeldetag: 02.03.2006
(51) Int. Cl.: B01J 35/00, B01J 23/22, B01J 27/198, C07C 51/265, C07C 51/31, C07C 63/16

(54) **VERFAHREN ZUR HERSTELLUNG VON PHTHALSÄUREANHYDRID UNTER VERWENDUNG EINES MEHRLAGEN - KATALYSATORS**
PROCESS FOR PRODUCING PHTHALIC ANHYDRIDE USING A MULTI-LAYER CATALYST
PROCÉDÉ POUR LA FABRICATION D'ANHYDRIDE D'ACIDE PHTALIQUE UTILISANT UN CATALYSEUR MULTICOUCHE

(30) Priorität: 02.03.2005 DE 102005009473
(43) Veröffentlichungstag der Anmeldung: 14.11.2007
(73) Patentinhaber: Süd-Chemie IP GmbH & Co. KG, 81925 München (DE)
(72) Erfinder: GÜCKEL, Christian, 85567 Grafing (DE); DIALER, Harald, 81545 München (DE); ESTENFELDER, Marvin, 76199 Karlsruhe (DE); PITSCHI, Werner, 83052 Bruckmühl (DE)
(74) Vertreter: Kuba, Stefan
(86) Internationale Anmeldenummer: PCT/EP2006/001915
(87) Internationale Veröffentlichungsnummer: WO 2006/092304

(56) Entgegenhaltungen:
- EP-A- 0 676 400
- WO-A-99/61433
- WO-A-03/070680
- WO-A-2005/115615
- WO-A-2005/115616
- DE-A1- 10 323 818
- US-A- 2 142 678

## Beschreibung

Die Erfindung betrifft die Verwendung eines MehrlagenKatalysators, d.h. eines Katalysator mit drei oder mehr unterschiedlichen Lagen (Schichten) zur Herstellung von Phthalsäureanhydrid (PSA) durch Gasphasenoxidation von o-Xylol und/oder Naphthalin.

Die großtechnische Produktion von Phthalsäureanhydrid wird durch die katalytische Gasphasenoxidation von o-Xylol und/oder Naphthalin erzielt. Zu diesem Zwecke wird ein für die Reaktion geeigneter Katalysator in einen Reaktor, vorzugsweise einen sogenannten Rohrbündelreaktor, in dem eine Vielzahl von Rohren parallel angeordnet sind, gefüllt, und von oben oder unten mit einem Gemisch aus dem (den) Kohlenwasserstoff(en) und einem sauerstoffhaltigen Gas, beispielsweise Luft, durchströmt. Aufgrund der starken Wärmebildung solcher Oxidationsreaktionen ist es nötig, die Reaktionsrohre zur Vermeidung von sogenannten Hotspots ("Heißen Flecken") mit einem Wärmeträgermedium zu umspülen und somit die entstandene Wärmemenge abzuführen. Diese Energie kann zur Produktion von Dampf genutzt werden. Als Wärmeträgermedium dient in der Regel eine Salzschmelze und hier vorzugsweise ein eutektisches Gemisch aus NaNO₂ und KNO₃.

Heutzutage werden für die Oxidation von o-Xylol und/oder Naphthalin zu Phthalsäureanhydrid Mehrlagenkatalysatorsysteme eingesetzt. Ziel dabei ist es, die Aktivität der einzelnen Katalysatorlagen dem Reaktionsverlauf entlang der Reaktorachse anzupassen. Dadurch ist es möglich, eine hohe Ausbeute am Wertprodukt PSA und gleichzeitig eine möglichst geringe Ausbeute an dem unerwünschten Zwischenprodukt Phthalid zu erzielen. Üblicherweise weist die 1. Lage (= die am nächsten zum Reaktoreingang gelegene Schicht) die geringste Aktivität auf, da im reaktoreingangsnahen Bereich die höchsten Eduktkonzentrationen und damit die größten Reaktionsgeschwindigkeiten auftreten. Durch die bei der chemischen Umsetzung frei werdende Wärme wird das Reaktionsgas aufgeheizt bis zu der Stelle, an der die durch Reaktion erzeugte Energie gerade so groß ist wie die an das Kühlmittel abgegebene Energie. Diese heißeste Stelle im Reaktionsrohr wird Hotspot genannt. Eine zu hohe Aktivität in der 1. Lage würde zu einem unkontrollierten Ansteigen der Hotspot - Temperatur führen, die üblicherweise zu einer Selektivitätsreduktion oder gar zu einem "runaway" führen kann.

Ein weiterer wesentlicher Aspekt, der bei der Auslegung der Aktivität der einzelnen Katalysatorlagen beachtet werden muß, ist die Position des Hotspots in der 1. Katalysatorlage. Da sich mit zunehmender Betriebszeit die Katalysatoraktivität verringert, verschiebt sich die Position des Hotspots immer weiter in Richtung Reaktorausgang. Dies kann sogar so weit führen, dass der Hotspot aus der 1. Lage in die 2. Lage oder sogar in eine noch später folgende Lage wandert. Häufig muss aufgrund der damit einhergehenden signifikant abfallenden PSA-Ausbeute in einem solchen Fall der Katalysator ausgetauscht werden, was zu hohen Betriebsausfällen führt.

US 2,142,678 beschreibt die Verwendung eines Mehrlagenkatalysators zur Herstellung von Phthalsäureanhydrid durch Gasphasenoxidation von Naphthalin.

EP 1 084 115 B1 beschreibt einen Mehrlagenkatalysator für die Oxidation von o-Xylol und/oder Naphthalin zu Phthalsäureanhydrid, bei dem die Aktivität der einzelnen Katalysatorlagen von der Reaktoreingangs- zur Reaktorausgangsseite kontinuierlich zunimmt. Dies wird erzielt durch Erhöhung der aktiven Masse in Kombination mit der Absenkung des Alkalimetallgehalts des Katalysators derart, dass die Katalysatorlage direkt am Katalysatoreingang den geringsten Aktivmassengehalt und höchsten Alkalimetallgehalt aufweist.

DE 103 23 818 A1 beschreibt einen Mehrlagenkatalysator für die Oxidation von o-Xylol und/oder Naphthalin zu Phthalsäureanhydrid aus mindestens drei aufeinander folgenden Schichten, bei dem die Aktivität der einzelnen Katalysatorlagen von der Reaktoreingangs- zur Reaktorausgangsseite kontinuierlich zunimmt. Dies wird erzielt durch Einsatz von TiO₂ mit unterschiedlicher BET-Oberfläche derart, dass die BET-Oberfläche des verwendeten TiO₂ in der Lage am Reaktoreingang geringer ist als in den nachfolgenden Lagen und in der letzten Lage (Reaktorausgang) am höchsten ist.

DE 103 23 461 A1 beschreibt einen Mehrlagenkatalysator für die Oxidation von o-Xylol und/oder Naphthalin zu Phthalsäureanhydrid, bei dem die Aktivität der einzelnen Katalysatorlagen von der Reaktoreingangs- zur Reaktorausgangsseite zunimmt, wobei das Verhältnis von V₂O₅ zu Sb₂O₃ in der ersten Lage zwischen 3,5 : 1 und 5 : 1 liegt.

DE 103 23 817 A1 beschreibt einen Mehrlagenkatalysator für die Oxidation von o-Xylol und/oder Naphthalin zu Phthalsäureanhydrid aus mindestens drei aufeinander folgenden Schichten, bei dem die Aktivität der einzelnen Katalysatorlagen von der Reaktoreingangs- zur Reaktorausgangsseite kontinuierlich zunimmt, wobei die letzte, dem Reaktorausgang am nächsten liegende Schicht mehr als 10 Gew.-% V₂O₅ enthält und als einzige Lage P aufweist.

Nachteilig an den dort angegebenen erfindungsgemäßen Katalysatoren ist, dass trotz des Einsatzes solcher strukturierter Katalysatoren die Lebensdauer des Katalysators insbesondere im Hinblick auf die zunehmende Verlagerung des Hotspots in der Richtung des Gasstroms nicht zufriedenstellend ist. Eine Positionierung des Hotspots in der (aktivsten) Katalysatorlage weiter zur Gasaustrittsseite hin beschränkt auch die Möglichkeit der Feineinstellung der Selektivität des Katalysators zur Vermeidung von unerwünschten Nebenprodukten.

Es besteht daher ein ständiger Bedarf nach verbesserten mehrlagigen (mehrschichtigen) Katalysatoren zur Herstellung von Phthalsäureanhydrid und anderen durch Partialoxidation von Kohlenwasserstoffen erhaltenen Produkten.

Eine Aufgabe der vorliegenden Erfindung war es daher, einen verbesserten Katalysator zur Herstellung von Phthalsäureanhydrid durch Gasphasenoxidation von o-Xylol und/oder Naphthalin bereitzustellen, der die Nachteile des Stand der Technik vermeidet und insbesondere eine vorteilhafte Positionierung des Hotspots und eine verbesserte Lebensdauer des Katalysators ermöglicht. Ziel der vorliegenden Erfindung ist es insbesondere, eine Erhöhung der Katalysatorlebensdauer bei gleich-bleibender oder sogar verbesserter Produkt-Ausbeute herbeizuführen.

Nach einem ersten Aspekt der Erfindung wird diese Aufgabe durch das Verfahren gemäß Anspruch 1 gelöst. Bevorzugte Ausführungsformen sind in den Unteransprüchen angegeben.

Dabei wurde überraschend gefunden, dass die erfindungsgemäße Aufgabe durch Verwendung bzw. Einführung einer ersten, sehr aktiven Katalysatorlage an der Reaktoreingangsseite (Gaseintrittsseite) gelöst werden kann. Durch diese direkt am Reaktoreingang befindliche erste Katalysatorlage mit einer höheren Aktivität als die der darauf folgende zweiten Katalysatorlage, wird die Reaktionsgeschwindigkeit in einem vergleichsweise kurzen Bereich am Reaktoreingang, in dem aufgrund der niedrigen Temperatur üblicherweise nur geringe Reaktionsgeschwindigkeiten und damit geringe chemische Umsetzungen auftreten, deutlich erhöht. Daraus resultiert letztlich eine frühere Positionierung des Hotspots näher am Reaktoreingang als ohne die erfindungsgemäße erste Katalysatorlage. Dies ist im Hinblick auf eine lange Lebensdauer (Standzeit) wie oben beschrieben von Vorteil und ermöglicht auch eine bessere Feineinstellung der Katalysatorselektivität in den Katalysatorabschnitten, die zur Gasaustrittsseite hin "hinter" dem o.g. Hotspot liegen. Dadurch lassen sich auch Ausbeute und Selektivität steigern.

Nach einem Aspekt betrifft die vorliegende Erfindung somit die Herstellung von Phthalsäureanhydrid durch Gasphasenoxidation von o-Xylol und/oder Naphthalin, wobei der Katalysator mindestens eine erste, zur Gaseintrittsseite hin gelegene Katalysatorlage, eine zweite, näher zur Gasaustrittsseite hin gelegene Katalysatorlage und eine dritte, noch näher zur oder an der Gasaustrittsseite hin gelegene Katalysatorlage enthält, und wobei die Katalysatorlagen unterschiedlich zusammengesetzt sind und jeweils eine Aktivmasse enthaltend TiO₂ aufweisen, dadurch gekennzeichnet, dass die Katalysatoraktivität der ersten Katalysatorlage höher ist als die Katalysatoraktivität der zweiten Katalysatorlage.

Erfindungsgemäß sind die einzelnen Katalysatorlagen unterschiedlich zusammengesetzt. Dabei können sich die einzelnen Lagen, insbesondere die erste und die zweite Katalysatorlage, auch nur durch einen unterschiedlichen Aktivmassegehalt unterscheiden, z.B. auch bezogen auf ein bestimmtes Reaktorvolumen.

Nach einer bevorzugten erfindungsgemäßen Ausführungsform weist der stückige Katalysator in den einzelnen Katalysatorlagen jeweils inerte keramische Träger und eine darauf im Fließbett unter Beihilfe von geeigneten Bindemitteln aufgebrachte Schicht mit katalytisch aktiven Metalloxiden auf.

Nach einer bevorzugten erfindungsgemäßen Ausführungsform ist die Aktivität der dritten Katalysatorlage höher als die der zweiten Katalysatorlage. Weiterhin bevorzugt ist die Aktivität einer vierten Katalysatorlage, soweit vorhanden, höher als die der dritten Katalysatorlage. Sofern eine fünfte Katalysatorlage vorhanden ist, liegt deren Aktivität wiederum vorzugsweise höher als die Aktivität der vierten Katalysatorlage. Es wurde weiterhin gefunden, dass es für die Performance und Lebensdauer des Katalysators besonders günstig ist, wenn dann die Aktivität von der 2. Lage bis zur Austrittsseite des Reaktionsgasgemisches, d.h. bis zur letzten Katalysatorlage kontinuierlich (d.h. von Katalysatorlage zu Katalysatorlage) zunimmt.

Erfindungsgemäß kann die Aktivität der ersten Katalysatorlage durch alle dem Fachmann geläufige Maßnahmen so eingestellt werden, dass sie höher liegt als die Aktivität der nachfolgenden zweiten Katalysatorlage.

Nach einer bevorzugten erfindungsgemäßen Ausführungsform kann die erhöhte Aktivität in der ersten Katalysatorlage beispielsweise erzielt werden durch:
- einen höheren Gehalt an aktiver Masse als in der 2. Lage
- eine höhere BET-Oberfläche (insbesondere des verwendeten TiO₂) als in der 2. Lage
- einen höheren Vanadiumgehalt als in der 2. Lage
- einen geringeren Cs-Gehalt als in der 2. Lage
- einen geringeren Sb-Gehalt als in der 2. Lage
- eine Erhöhung der Schüttdichte in der ersten Katalysatorlage, z.B. durch Einsatz einer anderen (Ring)geometrie des verwendeten inerten Formkörpers;
- die Gegenwart bzw. eine größere Menge anderer aktivitätssteigernder Promotoren als in der zweiten Katalysatorlage;
- die Abwesenheit bzw. eine geringere Menge an aktivitätsdämpfenden Promotoren als in der zweiten Katalysatorlage;
oder die Kombinationen von zwei oder mehr der vorstehenden Maßnahmen.

Besonders bevorzugt ist, dass die erste Katalysatorlage im Vergleich zur zweiten Katalysatorlage einen höheren Aktivmassegehalt und/oder eine höhere BET-Oberfläche aufweist. Da die BET-Oberfläche der Katalysatorlage in erster Linie von der BET-Oberfläche des verwendeten TiO₂ abhängt, ist nach einer bevorzugten erfindungsgemäßen Ausführungsform die BET-Oberfläche des TiO₂ in der ersten Katalysatorlage höher als die BET-Oberfläche des TiO₂ in der zweiten Katalysatorlage.

Die vorstehenden Maßnahmen zur Erhöhung der Aktivität der ersten Katalysatorlage gegenüber der zweiten Katalysatorlage können natürlich auch zur bevorzugten Abstimmung der Aktivitäten der nachfolgenden Katalysatorlagen (z.B. der dritten und vierten Katalysatorlage) eingesetzt werden.

Nach einer bevorzugten erfindungsgemäßen Ausführungsform liegt die Aktivität der ersten Katalysatorlage um mindestens 5 %, insbesondere mindestens 10 %, bevorzugt mindestens 20 %, insbesondere bevorzugt mindestens 30 % höher als die Aktivität der nachfolgenden zweiten Katalysatorlage. Eine Methode zur Bestimmung bzw. zum Vergleich der Aktivität von Katalysatoren (lagen) ist nachstehend im Methodenteil angegeben. Bevorzugt ist die zweite Katalysatorlage die am wenigsten aktive Katalysatorlage im gesamten Katalysator.

Erfindungsgemäß wird die Länge der ersten Katalysatorlage (1. Lage) vorzugsweise derart bemessen, dass der Hotspot unter den gewünschten Reaktionsbedingungen in der nachfolgenden zweiten Katalysatorlage (2. Lage) und nicht in der ersten Katalysatorlage selber auftritt. Somit liegt der Hotspot vorzugsweise in der zweiten Katalysatorlage, wodurch sich die vorstehenden Vorteile besonders gut verwirklichen lassen. Aus diesem Grunde ist eine bevorzugte Länge bei üblichen Reaktorrohren 20 - 70 cm, besonders bevorzugt 30 - 60 cm. Dabei liegt die übliche Länge der Reaktorrohre und der darin befindlichen Katalysatorschüttung zwischen etwa 2,5 und 3,5 m. Einfluss auf die Länge der ersten Katalysatorlage haben neben dem Volumenstrom und der Beladung insbesondere auch der axiale Temperaturgradient im umgebenden Kühlmittel (Salzbad). Bei einem hohen axialen Temperaturgradient, der bei schlechter Kühlmittelumwälzung entsteht, ist die Temperatur des Kühlmittels am Reaktoreingang um bis zu 10 °C höher als am Reaktorausgang. In diesem Falle ist die Länge der ersten Katalysatorlage kürzer und deren Aktivität moderater zu wählen als bei einem geringen axialen Temperaturgradient im Kühlmittel.

Nach einer bevorzugten erfindungsgemäßen Ausführungsform beträgt die Länge der 1. Lage bevorzugt 5 - 25 %, besonders bevorzugt 10 - 25 % der Gesamtlänge des Katalysators bzw. der Katalysatorschüttung. Neben anderen Faktoren spielt bei der Gestaltung der Länge auch die Höhe des axialen Temperaturgradienten im umgebenden Kühlmedium eine Rolle. In jeden Fall ist die Länge der 1. Lage kleiner, als es der Position eines fiktiven Hotspots, gemessen als Abstand von Beginn der Katalysatorschüttung bis zum Erreichen der max. Temperatur, entspräche, der sich ausbilden würde, wenn man anstelle der 1. Lage den entsprechenden Bereich auch mit Katalysator der 2. Lage füllen würde. Nach einer besonders bevorzugten Ausführungsform ist das Verhältnis der Länge der ersten Lage zur Länge der zweiten Lage kleiner oder gleich 0,9. Weiter bevorzugt liegt dieses Verhältnis zwischen etwa 0,1 und 0,9, insbesondere 0,1 und 0,7, vorzugsweise zwischen 0,15 und 0,5. Dadurch wird sichergestellt, dass die erste Lage nicht zu lang gegenüber der zweiten Lage, in der sich vorzugsweise der Hotspot befinden soll, ist.

Nach einer weiteren bevorzugten erfindungsgemäßen Ausführungsform enthalten die einzelnen Katalysatorlagen jeweils zumindest Titan und Vanadium in der Aktivmasse. Es wurde auch gefunden, dass besonders gute Ergebnisse bei der PSA-Herstellung erzielt werden, wenn der Vanadiumgehalt der aktiven Masse in der ersten Katalysatorlage, berechnet als V₂O₅, bei mehr als 4 Gew.-%, insbesondere bei mehr als 5 Gew.-% liegt.

Zudem weisen die einzelnen Katalysatorlagen vorzugsweise kein Molybdän und/oder Wolfram auf, insbesondere nicht in einem atomaren Verhältnis zu Vanadium im Bereich zwischen 0,01 und 2. Nach einer bevorzugten Ausführungsform wird weiterhin kein Ni oder Co in den eingesetzten Katalysatoren verwendet. Nach einer bevorzugten Ausführungsform liegt der Na-Gehalt in der aktiven Masse bei weniger als 500 ppm, insbesondere weniger als 450 ppm.

Weiter bevorzugt sind jeweils auch Cs und/oder Sb in den Katalysatorlagen enthalten. Nach einer besonders bevorzugten erfindungsgemäßen Ausführungsform weist zumindest die zweite Katalysatorlage Cs auf, wobei bevorzugt die erste Katalysatorlage einen geringeren Cs-Gehalt (oder gar kein Cs) aufweist. Es wurde gefunden, dass sich dadurch das Zusammenspiel der ersten Katalysatorlage mit einer gewünschten hohen Reaktionsgeschwindigkeit zur primären Umsetzung der Edukte, insbesondere von o-Xylol und/oder Naphthalin, unmittelbar am Beginn der Schüttung zur Gaseintrittsseite, und der zweiten Katalysatorlage mit einer frühen Positionierung des Hotspots näher am Reaktoreingang besonders gut bewerkstelligen lässt.

Bevorzugt liegen die Zusammensetzungsbereiche der eingesetzten Katalysatoren (Aktivmasse) in den einzelnen Lagen wie folgt:

| **Zusammensetzung** | **Bereiche** |
|---|---|
| V₂O₅ / Gew.% | 1 - 25 |
| Sb₂O₃ / Gew.% | 0 - 4 |
| Cs / Gew.% | 0 - 1 |
| P / Gew.% | 0 - 2 |
| BET TiO₂ / (m²/g) | 10 bis 50 |
| Anteil AM/Gew.% | 4 - 20, vorzugsweise 4 - 15 |

Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus TiO₂. Es wurde im Rahmen der vorliegenden Erfindung auch gefunden, dass sich besonders vorteilhafte Katalysatoren nach einer Ausführungsform herstellen lassen, wenn der Aktivmassegehalt von der zweiten Katalysatorlage zu der zur Gasaustrittsseite hin gelegenen Katalysatorlage abnimmt. Dabei weist nach einer bevorzugten Ausführungsform die zweite Katalysatorlage einen Aktivmassegehalt zwischen etwa 6 und 12 Gew.-%, insbesondere zwischen etwa 6 und 11 Gew.-%, die dritte Katalysatorlage einen Aktivmassegehalt zwischen etwa 5 und 11 Gew.-%, insbesondere zwischen etwa 6 und 10 Gew.-% und die vierte Katalysatorlage (soweit vorhanden) einen Aktivmassegehalt zwischen etwa 4 und 10 Gew.-%, insbesondere zwischen etwa 5 und 9 Gew.-%, auf. Es sind jedoch grundsätzlich auch solche Katalysatoren erfasst, bei denen der Aktivmassegehalt von der 2. Lage bis zur letzten Lage gleichbleibend oder zunehmend ist, d.h.: *Aktivmassegehalt*_{*2*.} *_{Lage}* ≤ *Aktivmassegehalt_{3. Lage}* ≤ ... ≤ *Aktivmassegehalt_{letzte Lage.}*

Nach einer vorteilhaften Ausführungsform liegt zumindest der Aktivmassegehalt der letzten Lage höher als derjenige der 2. Lage.

Nach einer besonders bevorzugten erfindungsgemäßen Ausführungsform weist der erfindungsgemäße Katalysator der ersten Katalysatorlage einen Aktivmassegehalt zwischen etwa 6 und 20 Gew.-%, bevorzugt zwischen etwa 7 und 15 Gew.-%, auf.

Die Ausdrücke erste, zweite, dritte bzw. vierte Katalysatorlage werden im Zusammenhang mit der vorliegenden Erfindung wie folgt verwendet: als erste Katalysatorlage wird die zur Gaseintrittsseite hin gelegene Katalysatorlage bezeichnet. Zur Gasaustrittsseite hin sind im erfindungsgemäßen Katalysator noch mindestens zwei weitere Katalysatorlagen enthalten, die als zweite, dritte bzw. vierte Katalysatorlage bezeichnet werden. Die dritte Katalysatorlage liegt dabei näher zur Gasaustrittsseite als die zweite Katalysatorlage. Die einzelnen Katalysatorlagen können mit oder ohne Durchmischung in den Grenzbereichen befüllt werden, um den (Mehrlagen)-Katalysator zu erhalten.

Nach einer besonders bevorzugten erfindungsgemäßen Ausführungsform weist der erfindungsgemäß verwendete Katalysator vier Katalysatorlagen auf. Dann liegt die vierte Katalysatorlage an der Gasaustrittsseite. Die Anwesenheit von zusätzlichen Katalysatorlagen gasstromab ist jedoch nicht ausgeschlossen. Beispielsweise kann nach einer erfindungsgemäßen Ausführungsform der vierten Katalysatorlage wie hierin definiert noch eine fünfte Katalysatorlage nachfolgen. Unabhängig davon ist bei der Phthalsäureanhydrid-Herstellung gegebenenfalls auch die Verwendung eines sogenannten Finishing-Reaktors möglich, wie er z.B. in der DE-A-198 07 018 oder der DE-A-20 05 969 beschrieben ist.

Nach einer bevorzugten erfindungsgemäßen Ausführungsform nimmt dabei die BET-Oberfläche des eingesetzten TiO₂ von der zweiten Katalysatorlage zur der zur Gasaustrittsseite hin gelegenen Katalysatorlage zu. Mit anderen Worten ist bevorzugt, dass die BET-Oberfläche des eingesetzten TiO₂ in der zweiten Katalysatorlage niedriger ist als die BET-Oberfläche des eingesetzten TiO₂ in der zur Gasaustrittsseite hin gelegenen (letzten) Katalysatorlage. Bevorzugte Bereiche für die BET-Oberfläche des TiO₂ sind 15 bis 30 m²/g für die mittleren Katalysatorlagen, und 15 bis 45 m²/g für die zur Gasaustrittsseite hin gelegene (letzte) Katalysatorlage. Besonders vorteilhafte Katalysatoren werden auch erhalten, wenn die BET-Oberflächen des TiO₂ der mittleren Katalysatorlagen gleich sind, während die BET-Oberfläche des TiO₂ in der letzten Katalysatorlage demgegenüber größer ist. Die BET-Oberfläche des TiO₂ der ersten Katalysatorlage ist vorzugsweise größer oder gleich der BET-Oberfläche des TiO₂ der zweiten oder der mittleren Katalysatorlagen und liegt insbesondere im Bereich von etwa 15 bis 45 m²/g. Nach einer erfindungsgemäßen Ausführungsform ist die BET-Oberfläche des eingesetzten TiO₂ wie folgt: BET_{TiO2, 2. Lage} ≤ BET_{TiO2, 3. Lage} ≤ ... ≤ BET_{TiO2, letzte Lage}. Noch Weiter bevorzugt ist BET_{TiO2, 1. Lage} ≥ BET_{TiO2, 2. Lage}.

Das Temperaturmanagement bei der Gasphasenoxidation von o-Xylol zu Phthalsäureanhydrid ist dem Fachmann aus dem Stand der Technik hinreichend bekannt, wobei beispielsweise auf die DE 100 40 827 A1 verwiesen werden kann.

Im Allgemeinen wird bei der Verwendung des erfindungsgemäßen Katalysators zur Herstellung von Phthalsäureanhydrid ein Gemisch aus einem molekularen Sauerstoff enthaltenden Gas, beispielsweise Luft, und dem zu oxidierenden Ausgangsmaterial (insbesondere o-Xylol und/oder Naphthalin) durch einen Festbettreaktor, insbesondere einen Rohrbündelreaktor, der aus einer Vielzahl parallel angeordneter Rohre bestehen kann, geleitet. In den Reaktorrohren befindet sich jeweils eine Schüttung aus mindestens einem Katalysator. Auf die Vorzüge einer Schüttung aus mehreren (unterschiedlichen) Katalysatorlagen wurde oben bereits eingegangen.

Beim Einsatz der hierin beschriebenen Katalysatoren zur Herstellung von Phthalsäureanhydrid durch Gasphasenoxidation von o-Xylol und/oder Naphthalin wurde überraschend festgestellt, dass mit den erfindungsgemäß verwendeten Katalysatoren sehr gute PSA-Ausbeuten bei sehr geringen Anteilen an Phthalid und einer Position des Hotspots nahe am Reaktoreingang erzielt werden, wodurch eine verbesserte Standzeit des Katalysators ermöglicht wird.

Nach einer bevorzugten erfindungsgemäßen Ausführungsform weist das eingesetzte TiO₂ (üblicherweise in der Anatas-Form) eine BET-Oberfläche von mindestens 15, vorzugsweise zwischen 15 und 60 m²/g, insbesondere zwischen etwa 15 und 45 m²/g und besonders bevorzugt zwischen 15 und 40 m²/g auf. Weiterhin wird bevorzugt, dass mindestens 30%, insbesondere mindestens 40% und bis zu 80%, bevorzugt bis zu 75%, insbesondere bis zu 70% des gesamten Porenvolumens des TiO₂ durch Poren mit einem Radius zwischen 60 und 400 nm gebildet werden. Die Bestimmung der hierin angegebenen Porenvolumina bzw. -anteile erfolgt, soweit nicht anders angegeben, mittels Quecksilberporosimetrie (gemäß DIN 66133). Die Angabe des Gesamtporenvolumens bezieht sich dabei in der vorliegenden Beschreibung jeweils auf das gesamte mittels Quecksilberporosimetrie gemessene Porenvolumen zwischen 7500 und 3,7 nm Porenradiengröße. Poren mit einem Radius von mehr als 400 nm stellen bevorzugt weniger als etwa 30%, insbesondere weniger als etwa 22%, besonders bevorzugt weniger als 20% des gesamten Porenvolumens des eingesetzten TiO₂ dar. Weiterhin bevorzugt wird, dass etwa 50 bis 75%, insbesondere etwa 50 bis 70%, besonders bevorzugt 50 bis 65% des gesamten Porenvolumens des TiO₂ durch Poren mit einem Radius von 60 bis 400 nm, und vorzugsweise etwa 15 bis 25% des gesamten Porenvolumens durch Poren mit einem Radius von mehr als 400 nm gebildet werden. Bezüglich der kleineren Porenradien wird bevorzugt, dass weniger als 30%, insbesondere weniger als 20% des gesamten Porenvolumens des TiO₂ durch Poren mit einem Radius von 3,7 bis 60 nm gebildet werden. Ein hier besonders bevorzugter Bereich beträgt für diese Porengröße etwa 10 bis 30% des gesamten Porenvolumens, insbesondere 12 bis 20%.

Nach einer weiteren bevorzugten Ausführungsform weist das eingesetzte TiO₂ die folgende Partikelgrößenverteilung auf: Der D₁₀-Wert liegt vorzugsweise bei 0,5 *µ*m oder darunter; der D₅₀-Wert (d.h. der Wert, bei dem jeweils die Hälfte der Partikel einen größeren bzw. kleineren Partikeldurchmesser aufweist) liegt vorzugsweise bei 1,5 *µ*m oder darunter; der D₉₀-Wert liegt vorzugsweise bei 4 *µ*m oder darunter. Bevorzugt liegt der D₉₀-Wert des eingesetzten TiO₂ zwischen etwa 0,5 und 20 *µ*m, insbesondere zwischen etwa 1 und 10 *µ*m, besonders bevorzugt zwischen etwa 2 und 5 *µ*m. In elektronenmikroskopischen Aufnahmen weist das erfindungsgemäß eingesetzte TiO₂ bevorzugt eine offenporige, schwammartige Struktur auf, wobei Primärteilchen oder -kristallite zu mehr als 30%, insbesondere mehr als 50%, zu offenporigen Agglomeraten zusammengeschlossen sind. Es wird angenommen, ohne dass die Erfindung auf diese Annahme beschränkt wäre, dass durch diese besondere Struktur des eingesetzten TiO₂, die sich in der Porenradienverteilung spiegelt, besonders günstige Reaktionsbedingungen für die Gasphasenoxidation geschaffen werden.

Grundsätzlich kann in dem erfindungsgemäßen Katalysator auch ein anderes Titandioxid mit einer anderen Spezifikation als vorstehend beschrieben, d.h. einer anderen BET-Oberfläche, Porosimetrie und/oder Partikelgrößenverteilung, verwendet werden. Erfindungsgemäß wird besonders bevorzugt, dass mindestens 50%, insbesondere mindestens 75%, besonders bevorzugt das gesamte verwendete TiO₂ eine BET-Oberfläche und Porosimetrie wie hierin definiert, und vorzugsweise auch die beschriebene Partikelgrößenverteilung aufweist. Dabei können auch Abmischungen verschiedener TiO₂-Materialien verwendet werden.

Je nach vorgesehener Verwendung des Katalysators können neben TiO₂ die dem Fachmann geläufigen und üblichen Komponenten in der aktiven Masse des Katalysators enthalten sein. Auch die Form des Katalysators bzw. dessen homogener oder heterogener Aufbau ist im Sinne der vorliegenden Erfindung grundsätzlich nicht beschränkt und kann jegliche dem Fachmann geläufige und für das jeweilige Anwendungsgebiet geeignet erscheinende Ausführungsform umfassen.

Zur Herstellung von Phthalsäureanhydrid haben sich insbesondere sogenannte Schalenkatalysatoren bewährt. Hierbei wird ein unter den Reaktionsbedingungen inerter Träger, beispielsweise aus Quarz (SiO₂), Porzellan, Magnesiumoxid, Zinndioxid, Siliciumcarbid, Rutil, Tonerde (Al₂O₃), Aluminiumsilicat, Magnesiumsilicat (Steatit), Zirkoniumsilicat oder Cersilicat, oder aus Mischungen der vorstehenden Materialien verwendet. Der Träger kann beispielsweise die Form von Ringen, Kugeln, Schalen oder Hohlzylindern aufweisen. Darauf wird in verhältnismäßig dünnen Schichten (Schalen) die katalytisch aktive Masse aufgebracht. Es können auch zwei oder mehrere Schichten der gleichen oder unterschiedlich zusammengesetzter katalytisch aktiver Masse aufgebracht werden.

Bezüglich der weiteren Komponenten der katalytisch aktiven Masse des erfindungsgemäßen Katalysators (neben TiO₂) kann grundsätzlich auf die im einschlägigen Stand der Technik beschriebenen und dem Fachmann geläufigen Zusammensetzungen bzw. Komponenten verwiesen werden. Dabei handelt es sich hauptsächlich um Katalysatorsysteme, die neben Titanoxid(en) Oxide des Vanadiums enthalten. Solche Katalysatoren sind z.B. in der EP 0 964 744 B1 beschrieben, deren diesbezügliche Offenbarung hiermit ausdrücklich durch Inbezugnahme in die Beschreibung aufgenommen wird. In vielen Fällen kann es bevorzugt sein, dass für die einzelnen Katalysatorlagen des erfindungsgemäßen Katalysators ein V₂O_{B}-Material mit recht kleiner Teilchengröße verwendet wird, um das Aufspriten auf das TiO₂ zu begünstigen. Beispielsweise können mindestens 90 % der eingesetzten V₂O₅-Teilchen einen Durchmesser von 20 *µ*m oder weniger aufweisen. Hier kann z.B. auf die DE 10344846 A1 verwiesen werden.

Insbesondere sind im Stand der Technik eine Reihe von Promotoren zur Steigerung der Produktivität der Katalysatoren beschrieben, die im erfindungsgemäßen Katalysator ebenfalls eingesetzt werden können. Dazu gehören u.a. die Alkali- und Erdalkalimetalle, Thallium, Antimon, Phosphor, Eisen, Niob, Kobalt, Molybdän, Silber, Wolfram, Zinn, Blei und/oder Bismut sowie Mischungen aus zwei oder mehreren der vorstehenden Komponenten. Nach einer bevorzugten erfindungsgemäßen Ausführungsform enthalten die erfindungsgemäß eingesetzten Katalysatoren somit ein oder mehrere der vorstehenden Promotoren. Beispielsweise ist in der DE 21 59 441 A ein Katalysator beschrieben, der neben Titandioxid der Anatas-Modifikation aus 1 bis 30 Gew.-% Vanadiumpentoxid und Zirkondioxid besteht. Eine Aufzählung geeigneter Promotoren findet sich auch in der WO2004/103561, Seite 5, Zeilen 29 bis 37, auf die ebenfalls verwiesen wird. Über die einzelnen Promotoren lässt sich die Aktivität und Selektivität der Katalysatoren beeinflussen, insbesondere durch Absenkung oder Erhöhung der Aktivität. Zu den die Selektivität erhöhenden Promotoren zählen beispielsweise die Alkalimetalloxide und oxidische Phosphorverbindungen, insbesondere Phosphorpentoxid. Nach einer bevorzugten Ausführungsform enthält die erste Katalysatorlage, und bevorzugt auch die zweite Katalysatorlage keinen Phosphor. Es wurde gefunden, dass dadurch eine hohe Aktivität erreicht werden kann, wobei die Selektivität in den nachfolgenden Katalysatorlagen (3. und folgende Lage(n)) z.B. durch die Anwesenheit von Phosphor vorteilhaft eingestellt werden kann. In einigen Fällen kann es vorteilhaft sein, wenn nur die letzte Lage Phosphor aufweist. Nach einer weiteren bevorzugten Ausführungsform liegt das Verhältnis von Vanadium, berechnet als V₂O₃, und Antimon, berechnet als Sb₂O₃, im Katalysator der 1. Lage und/oder im Katalysator der 2. Lage zwischen etwa 3,5 : 1 und 5 : 1, wie beispielsweise in der DE 103 23 461 A beschrieben.

Nach einer weiteren bevorzugten Ausführungsform ist der Alkaligehalt, bevorzugt der Cs-Gehalt, im erfindungsgemäßen Katalysator gleichbleibend oder abnehmend von der 2. Lage bis zur letzten Lage (an der Gasaustrittsseite). Mit anderen Worten gilt: Cs-Gehalt_{2. Lage} ≥ Cs-Gehalt_{3. Lage} ≥... ≥ Cs-Gehalt_{letzte Lage}. Besonders bevorzugt weist die letzte Katalysatorlage kein Cs auf.

Zur Herstellung der erfindungsgemäßen Katalysatoren sind im Stand der Technik zahlreiche geeignete Verfahren beschrieben, so dass eine detaillierte Darstellung hier grundsätzlich nicht erforderlich ist. Zur Herstellung von Schalenkatalysatoren kann beispielsweise auf das in der DE-A-16 42 938 oder der DE-A 17 69 998 beschriebene Verfahren verwiesen werden, worin eine ein wässriges und/oder ein organisches Lösungsmittel enthaltende Lösung oder Suspension der Komponenten der katalytisch aktiven Masse und/oder deren Vorläuferverbindungen (häufig als "Maische" bezeichnet) auf das Trägermaterial in einer beheizten Dragiertrommel bei erhöhter Temperatur aufgesprüht wird, bis der gewünschte Gehalt an katalytisch aktiver Masse, bezogen auf das Katalysatorgesamtgewicht, erreicht ist. Auch lässt sich gemäß der DE 21 06 796 die Aufbringung (Beschichtung) der katalytisch aktiven Masse auf den inerten Träger in Wirbelbeschichtern durchführen.

Bevorzugt werden sogenannte Schalenkatalysatoren durch das Aufbringen einer dünnen Schicht von 50 bis 500 *µ*m der Aktivkomponenten auf einen inerten Träger hergestellt (z.B. US 2,035,606). Als Träger haben sich insbesondere Kugeln oder Hohlzylinder bewährt. Diese Formkörper ergeben eine hohe Packungsdichte bei niedrigem Druckverlust und verringern die Gefahr der Bildung von Packungsfehlern beim Einfüllen des Katalysators in die Reaktionsrohre.

Die geschmolzenen und gesinterten Formkörper müssen innerhalb des Temperaturbereiches der ablaufenden Reaktion hitzebeständig sein. Wie vorstehend ausgeführt, kommen dabei beispielsweise Siliciumcarbid, Steatit, Quarz, Porzellan, SiO₂, Al₂O₂ oder Tonerde in Frage.

Der Vorteil der Beschichtung von Trägerkörpern im Wirbelbett ist die hohe Gleichmäßigkeit der Schichtdicke, die für die katalytische Leistung des Katalysators eine entscheidende Rolle spielt. Eine besonders gleichmäßige Beschichtung erhält man durch Aufsprühen einer Suspension oder Lösung der Aktivkomponenten auf den erwärmten Träger bei 80 bis 200°C im Wirbelbett, beispielsweise gemäß DE 12 80 756, DE 198 28 583 oder DE 197 09 589. Im Gegensatz zu der Beschichtung in Dragiertrommeln kann bei Verwendung von Hohlzylindern als Träger in den genannten Wirbelbettverfahren auch die Innenseite der Hohlzylinder gleichmäßig beschichtet werden. Unter den oben genannten Wirbelbettverfahren ist insbesondere das Verfahren nach DE 197 09 589 von Vorteil, da durch die überwiegend horizontale, kreisförmige Bewegung der Träger neben einer gleichmäßigen Beschichtung auch eine geringe Abrasion von Apparateteilen erreicht wird.

Für den Beschichtungsvorgang wird die wässrige Lösung oder Suspension der Aktivkomponenten und eines organischen Binders, vorzugsweise einem Copolymer aus Vinylacetat/Vinyllaurat, Vinylacetat/Ethylen oder Styrol/Acrylat, über eine oder mehrere Düsen auf den erwärmten, fluidisierten Träger aufgesprüht. Besonders günstig ist es, die Sprühflüssigkeit am Ort der höchsten Produktgeschwindigkeit aufzugeben, wodurch sich der Sprühstoff gleichmäßig im Bett verteilen kann. Der Sprühvorgang wird solange fortgeführt, bis entweder die Suspension verbraucht oder die erforderliche Menge an Aktivkomponenten auf dem Träger aufgebracht ist.

Nach einer besonders bevorzugten erfindungsgemäßen Ausführungsform wird die katalytisch aktive Masse des erfindungsgemäßen Katalysators im Fließbett oder Wirbelbett unter Beihilfe geeigneter Bindemittel aufgebracht, so dass ein Schalenkatalysator erzeugt wird. Geeignete Bindemittel umfassen dem Fachmann geläufige organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat sowie Vinylacetat/Ethylen. Besonders bevorzugt wird ein organischer polymerer oder copolymerer Kleber, insbesondere ein Vinylacetat-Copolymer-Kleber, als Bindemittel verwendet. Das verwendete Bindemittel wird in üblichen Mengen der katalytisch aktiven Masse zugegeben, beispielsweise mit etwa 10 bis 20 Gew.-%, bezogen auf den Feststoffgehalt der katalytisch aktiven Masse. Beispielsweise kann auf die EP 744 214 verwiesen werden. Soweit die Aufbringung der katalytisch aktiven Masse bei erhöhten Temperaturen von etwa 150°C erfolgt, ist, wie aus dem Stand der Technik bekannt, eine Aufbringung auf den Träger auch ohne organische Bindemittel möglich. Brauchbare Beschichtungstemperaturen bei Verwendung der vorstehend angegebenen Bindemittel liegen gemäß DE 21 06 796 beispielsweise zwischen etwa 50 und 450°C. Die verwendeten Bindemittel brennen beim Ausheizen des Katalysators bei Inbetriebnahme des gefüllten Reaktors innerhalb kurzer Zeit aus. Die Bindemittel dienen in erster Linie der Verstärkung der Haftung der katalytisch aktiven Masse auf dem Träger und der Verringerung von Abrieb beim Transport und Einfüllen des Katalysators.

Weitere mögliche Verfahren zur Herstellung von Schalenkatalysatoren für die katalytische Gasphasenoxidation von aromatischen Kohlenwasserstoffen zu Carbonsäuren und/oder Carbonsäureanhydriden sind beispielsweise in der WO 98/00778 bzw. EP-A 714 700 beschrieben worden. Danach wird aus einer Lösung und/oder einer Suspension der katalytisch aktiven Metalloxide und/oder deren Vorläuferverbindungen, gegebenenfalls in Anwesenheit von Hilfsmitteln für die Katalysatorherstellung, zunächst ein Pulver hergestellt, das anschließend für die Katalysatorherstellung auf dem Träger, gegebenenfalls nach Konditionierung sowie gegebenenfalls nach Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide oder einer Behandlung zur Entfernung flüchtiger Bestandteile unterzogen, wird.

Geeignete Bedingungen zur Durchführung eines Verfahrens zur Herstellung von Phthalsäureanhydrid aus o-Xylol und/oder Naphthalin sind dem Fachmann gleichermaßen aus dem Stand der Technik geläufig. Insbesondere wird auf die zusammenfassende Darstellung in K. Towae, W. Enke, R. Jäckh, N. Bhargana "Phthalic Acid and Derivatives" in Ullmann's Encyclopedia of Industrial Chemistry Vol. A. 20, 1992, 181 verwiesen und diese hiermit durch Bezugnahme aufgenommen. Beispielsweise können für den stationären Betriebszustand der Oxidation die aus der vorstehenden Literaturstelle der WO-A 98/37967 oder der WO 99/61433 bekannten Randbedingungen gewählt werden.

Dazu werden zunächst die Katalysatoren in die Reaktionsrohre des Reaktors, die von außen auf die Reaktionstemperatur, beispielsweise mittels Salzschmelzen, thermostatisiert sind, gefüllt. Über die so bereitete Katalysatorschüttung wird das Reaktionsgas bei Temperaturen von im Allgemeinen 300 bis 450°C, vorzugsweise 320 bis 420°C, und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von im Allgemeinen 750 bis 5000 h⁻¹ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel wie Dampf, Kohlendioxid und/oder Stickstoff enthalten kann, mit dem zu oxidierenden, aromatischen Kohlenwasserstoff erzeugt, wobei das den molekularen Sauerstoff enthaltende Gas im Allgemeinen 1 bis 100, vorzugsweise 2 bis 50 und besonders bevorzugt 10 bis 30 mol-% Sauerstoff, 0 bis 30, vorzugsweise 0 bis 10 mol-% Wasserdampf sowie 0 bis 50, vorzugsweise 0 bis 1 mol-% Kohlendioxid, Rest Stickstoff, enthalten kann. Zur Erzeugung des Reaktionsgases wird das den molekularen Sauerstoff enthaltende Gas im Allgemeinen mit 30 bis 150 g je Nm³ Gas des zu oxidierenden, aromatischen Kohlenwasserstoffs beschickt.

Nach einer besonders bevorzugten erfindungsgemäßen Ausführungsform enthält die Aktivmasse (katalytisch aktive Masse) des Katalysators der ersten Katalysatorlage zwischen 5 bis 16 Gew.-% V₂O₃, 0 bis 5 Gew.-% Sb₂O₃, 0,2 bis 0,75 Gew.-% Cs, 0 bis 3 Gew.-% Nb₂O₅, 0 bis 1 Gew.-% P. Der Rest der Aktivmasse besteht zu mindestens 90 Gew.-%, vorzugsweise mindestens 95 Gew-%, weiter bevorzugt mindestens 98 Gew.-%, insbesondere mindestens 99 Gew-%, weiter bevorzugt mindestens 99,5 Gew.-%, insbesondere 100 Gew.-% aus TiO₂. Nach einer besonders bevorzugten erfindungsgemäßen Ausführungsform liegt dabei die BET-Oberfläche des TiO₂ zwischen 15 und etwa 45 m²/g. Weiterhin wird bevorzugt, dass eine solche erste Katalysatorlage einen Längenanteil von 5 - 25 %, besonders bevorzugt 10 - 25 % an der Gesamtlänge aller vorhandenen Katalysatorlagen (Gesamtlänge des vorhandenen Katalysatorbettes) aufweist.

Nach einer besonders bevorzugten erfindungsgemäßen Ausführungsform enthält die Aktivmasse des Katalysators der zweiten Katalysatorlage zwischen 5 bis 15 Gew.-% V₂O₃, 0 bis 5 Gew.-Sb₂O₃, 0,2 bis 0,75 Gew.-% Cs, 0 bis 2 Gew.-% Nb₂O₅ und 0 bis 1 Gew.-% P. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew-%, weiter bevorzugt mindestens 99,5 Gew.-%, insbesondere 100 Gew.-% aus TiO₂. Nach einer besonders bevorzugten erfindungsgemäßen Ausführungsform liegt dabei die BET-Oberfläche des TiO₂ zwischen 15 und etwa 25 m²/g. Weiterhin wird bevorzugt, dass eine solche zweite Katalysatorlage einen Längenanteil von etwa 15 bis 60, insbesondere 20 bis 60% oder 20 bis 50% an der Gesamtlänge aller vorhandenen Katalysatorlagen (Gesamtlänge des vorhandenen Katalysatorbettes) aufweist.

Nach einer besonders bevorzugten erfindungsgemäßen Ausführungsform enthält die Aktivmasse des Katalysators der dritten Katalysatorlage 5 bis 15 Gew.-% V₂O₃, 0 bis 4 Gew.-% Sb₂O₃, 0,05 bis 0,5 Gew.-% Cs, 0 bis 2 Gew.-% Nb₂O₅, 0- 1 Gew.-% P. Der Rest der Aktivmasse besteht zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus TiO₂. Dabei wird bevorzugt, dass das TiO₂ eine BET-Oberfläche zwischen etwa 15 und 25 m²/g aufweist. Weiterhin wird bevorzugt, dass diese dritte Lage einen Längenanteil von etwa 10 bis 30% der Gesamtlänge aller vorhandenen Katalysatorlagen einnimmt, insbesondere sofern sich an die dritte Lage noch mindestens eine weitere Katalysatorlage anschließt. Handelt es sich bei der dritten Lage um die letzte, also die dem Reaktorausgang am nächsten gelegene Lage, so ist ein Längenanteil für die 3. Lage von 20 - 50 % bevorzugt.

Nach einer besonders bevorzugten erfindungsgemäßen Ausführungsform enthält die Aktivmasse des Katalysators der vierten Katalysatorlage 5 bis 25 Gew.-% V₂O₃, 0 bis 5 Gew.-% Sb₂O₃, 0 bis 0,2 Gew.-% Cs, 0 bis 2 % P, 0 bis 1 Gew.-% Nb₂O₅. Der Rest der Aktivmasse besteht zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus TiO₂. Soweit die vierte Lage die an der Gasaustrittseite des Reaktors gelegene (letzte) Katalysatorlage darstellt, wird dabei eine BET-Oberfläche des TiO₂ bevorzugt, die etwas höher liegt als diejenige der näher zur Gaseintrittsseite hin gelegenen Schichten, insbesondere im Bereich zwischen etwa 15 bis etwa 45 m²/g. Weiterhin wird bevorzugt, dass eine solche vierte Katalysatorlage einen Längenanteil von etwa 10 bis 50%, insbesondere bevorzugt 10 bis 40 % der Gesamtlänge aller vorhandenen Katalysatorlagen einnimmt. Eine fünfte Katalysatorlage ist dann in der Regel nicht erforderlich, jedoch möglich.

Auch.wurde gefunden, dass nach einer bevorzugten Ausführungsform erfindungsgemäß verwendete Katalysatoren, die in den mittleren und gegebenenfalls in der ersten Katalysatorlage kein Phosphor in der katalytisch aktiven Masse aufweisen, besonders gute Aktivitäten bei gleichzeitig sehr hoher Selektivität ermöglichen. Es wird weiterhin bevorzugt, dass mindestens 0,05 Gew.-% der katalytisch aktiven Masse in der ersten und den mittleren Katalysatorlagen durch mindestens ein Alkalimetall, berechnet als Alkalimetall(e), gebildet wird. Besonders bevorzugt wird als Alkalimetall Cäsium.

Die erfindungsgemäß verwendeten Katalysatoren werden in üblicher Weise vor dem Einsatz temperaturbehandelt bzw. calciniert (konditioniert). Dabei hat sich als vorteilhaft herausgestellt, wenn der Katalysator mindestens 24 Stunden bei mindestens 390°C, insbesondere zwischen 24 und 72 Stunden bei ≥ 400°C, in einem O₂-haltigen Gas, insbesondere in Luft, calciniert wird. Die Temperatur sollte vorzugsweise 500°C, insbesondere 470°C, nicht überschreiten. Grundsätzlich sind jedoch auch andere Calcinierungsbedingungen, die dem Fachmann als geeignet erscheinen, nicht ausgeschlossen.

Auch betrifft die vorliegende Beschreibung ein Verfahren zur Herstellung eines Katalysators wie hierin beschrieben, umfassend die folgenden Schritte:
a. Bereitstellen einer katalytisch aktiven Masse wie hierin definiert,
b. Bereitstellen eines inerten Trägers, insbesondere eines inerten Trägerformkörpers;
c. Aufbringen der katalytisch aktiven Masse auf den inerten Träger, insbesondere in einer Wirbelschicht oder einem Fließbett.

Anschließend werden die einzelnen Katalysatoren in der gewünschten Reihenfolge als Katalysatorlagen in den Reaktor gefüllt, um den Mehrlagenkatalysator zu erhalten.

### METHODEN

Zur Bestimmung der Parameter der Katalysatoren werden die nachstehenden Methoden eingesetzt:

### 1. BET-Oberfläche:

Die Bestimmung erfolgt nach der BET-Methode gemäß DIN 66131; eine Veröffentlichung der BET-Methode findet sich auch in J. Am. Chem. Soc. 60, 309 (1938).

### 2. Porenradienverteilung:

Die Bestimmung der Porenradienverteilung des eingesetzten TiO₂ erfolgte durch Quecksilberporosimetrie gemäß DIN 66133; maximaler Druck: 2.000 bar, Porosimeter 4000 (Firma Porotec, DE), nach Angaben des Herstellers.

### 3. Partikelgrößen:

Die Bestimmung der Partikelgrößen erfolgte nach der Laserbeugungsmethode mit einem Fritsch Particle Sizer Analysette 22 Economy (Fa. Fritsch, DE) nach den Angaben des Herstellers, auch bezüglich der Probenvorbehandlung: die Probe wird in deionisiertem Wasser ohne Zusatz von Hilfsmitteln homogenisiert und 5 Minuten mit Ultraschall behandelt.

Die Bestimmung der BET-Oberfläche, der Porenradienverteilung bzw. des Porenvolumens sowie der Partikelgrößenverteilung erfolgte bezüglich des Titandioxids jeweils an dem bei 150°C im Vakuum getrockneten, uncalcinierten Material.

Auch die Angaben in der vorliegenden Beschreibung bezüglich der BET-Oberflächen der Katalysatoren bzw. Katalysatorlagen beziehen sich auf die BET-Oberflächen des jeweils eingesetzten TiO₂-Materials (getrocknet in Vakuum bei 150°C, uncalciniert, vgl. oben).

In der Regel wird die BET-Oberfläche des Katalysators durch die BET-Oberfläche des eingesetzten TiO₂ bestimmt, wobei durch den Zusatz weiterer katalytisch aktiver Komponenten die BET-Oberfläche in gewissem Umfang verändert wird. Dies ist dem Fachmann geläufig.

Der Aktivmasseanteil (Anteil der katalytisch aktiven Masse, ohne Bindemittel) bezieht sich jeweils auf den Anteil (in Gew.-%) der katalytisch aktiven Masse an dem Gesamtgewicht des Katalysators einschließlich Träger in der jeweiligen Katalysatorlage, gemessen nach Konditionierung über 4h bei 400°C in Luft.

### 4. Katalysatoraktivität:

Unter Aktivität des Katalysators in einer Katalysatorlage wird erfindungsgemäß die Fähigkeit des Katalysators verstanden, innerhalb eines definierten Volumens (= Bilanzraum), beispielsweise eines Reaktionsrohres definierter Länge und Innendurchmessers (z.B. 25 mm Innendurchmesser, 1 m Länge), bei vorgegebenen Reaktionsbedingungen (Temperatur, Druck, Konzentration, Verweilzeit) das eingesetzte Edukt umzusetzen. Der betrachtete Katalysator hat demgemäß dann eine höhere Aktivität als ein anderer Katalysator, wenn er in diesem vorgegebenen Volumen und unter den jeweils gleichen Reaktionsbedingungen einen höheren Eduktumsatz erzielt. Im Falle von o-Xylol bzw. Naphtalin als Edukt bemisst sich die Katalysatoraktivität somit anhand der Höhe des Umsatzes von o-Xylol bzw. Naphtalin zu den Oxidationsprodukten. Ursache für eine höhere Katalysatoraktivität kann entweder eine für die gewünschte Umsetzung optimierte Natur / Qualität der aktiven Zentren (vgl. bspw. "turn over frequency") oder eine erhöhte Anzahl von aktiven Zentren im gleichen Bilanzraum sein, was beispielsweise dann gegeben ist, wenn eine höhere Katalysatormasse mit sonst identischen Eigenschaften in dem Bilanzraum zugegen ist.

### Operationelle Quantifizierung der Aktivität:

Erfindungsgemäß ist die Aktivität der 1. Lage höher als die der 2. Lage. Dies bedeutet zunächst, dass - gemäß der vorstehenden Ausführung - am Ende eines mit "Lage 1 Katalysator" gefüllten und mit dem Eduktgemisch durchströmten Reaktionsraumes (= Reaktionsrohr definierter Länge und Innendurchmesser, z.B. 25 mm Innendurchmesser, 1 m Länge) ein höherer Eduktumsatz vorliegt als bei einem sonst identisch durchgeführten Vergleichsexperiment, bei dem der identische Reaktionsraum mit "Lage 2 Katalysator" gefüllt wurde.

Für einen solchen Test werden zweckmäßigerweise Bedingungen innerhalb der nachfolgend angegebenen Bereiche gewählt:

| | |
|---|---|
| Länge Reaktionsrohr: | 1 m |
| Innendurchmesser Reaktionsrohr: | 25 mm |
| Temperatur Kühlmedium: | 380 - 420 °C |
| Druck: | 1 - 1,5 bar absolut |
| o-Xylol Beladung Eduktgemisch: | 60 g o-Xylol/Nm³ Luft |

Die Quantifizierung der Aktivität der ersten Katalysatorlage im Vergleich zur Aktivität der zweiten Katalysatorlage lässt sich dann wie folgt anhand der nachstehenden erfindungsgemäßen Definition eines für Lage 1 verwendeten "Katalysators mit 10 % höherer Aktivität" gegenüber einem für Lage 2 verwendeten Katalysators bestimmen:
Der Vergleichskatalysator (= Lage 2 Katalysator mit der vorgesehenen Zusammensetzung) wird unter den oben angegebenen Bedingungen mit dem Eduktgemisch durchströmt, wobei der Gesamtvolumenstrom durch das Reaktionsrohr so eingestellt wird, dass der o-Xylolumsatz nach Durchströmen des Reaktionsraumes möglichst nahe an 50 % liegt.

In einem zweiten Experiment wird das gleiche Reaktionsvolumen mit Lage 1 (Test-)Katalysator gefüllt, der sich vom Lage 2 Katalysator nur dadurch unterscheidet, dass der Aktivmassenanteil um 10 % höher ist. Im Reaktionsvolumen befindet sich also 10 % mehr aktive Masse als im Falle des Vergleichskatalysators. Es wird dann unter den gleichen Reaktionsbedingungen der o-Xylolumsatz nach Durchströmen des mit Lage 1 Katalysator gefüllten Reaktionsraumes ermittelt. Dieser ist höher als mit dem Vergleichskatalysator, also höher als 50 %. Die Differenz zwischen dem so erhaltenen o-Xylolumsatz zu den 50 % Umsatz des Vergleichskatalysators wird als relative Maßzahl verwendet, die einer 10 %-igen Aktivitätserhöhung entspricht. Dabei ist es unerheblich, durch welche Änderung am Katalysator ein solcher Effekt erzielt wird. Entsprechend kann z.B. mit einem Katalysator, der sich vom vorgesehenen Lage 2 Katalysator nur dadurch unterscheidet, dass der Aktivmassenanteil um 20 % höher ist, eine Maßzahl für eine um 20% erhöhte Aktivität des Katalysators ermittelt werden etc..

Als Hotspot wird in der vorstehenden Beschreibung die maximal gemessene Temperatur im gesamten Katalysatorbett bezeichnet. Desweiteren bestehen noch (Neben-)Hotspots, d.h. maximale Temperaturen jeweils in den weiteren betrachteten Katalysatorlagen.

Die Erfindung wird nun anhand der nachstehenden, nicht beschränkenden Beispiele näher erläutert:

### BEISPIELE

### Beispiel 1: (Vergleichsbeispiel):

Ein 3 Lagen-Katalysatorsystem mit nachfolgender Zusammensetzung und Lagenlänge wurde in einen salzbadgekühlten Rohrreaktor mit 25 mm inneren Durchmesser gefüllt. Im Reaktionsrohr befand sich zentrisch angeordnet eine 3 mm Thermohülse mit eingebautem Zugelement zur Temperaturmessung. Durch das Rohr wurden stündlich von oben nach unten 4 Nm³ Luft mit einer Beladung von 30 - 100 g o-Xylol / Nm³ Luft (Reinheit o-Xylol > 99 %) an und bei einem Gesamtdruck von ca. 1450 mbar geleitet.

Bei einer Beladung von 60 - 65 g o-Xylol / Nm³ Luft wurde bei Salzbadtemperaturen zwischen 370 und 375 °C der Hotspot in der Lage 1 bei einer Position von 90 - 100 cm gemessen (vom Beginn der Schüttung in Richtung Reaktorausgang).

| Zusammensetzung | Lage 1 | Lage 2 | Lage 3 |
|---|---|---|---|
| | Länge: 150 cm | Länge: 60 cm | Länge: 80 cm |
| V₂O₅ / Gew. % | 7,5 | 7,5 | 7,5 |
| Sb₂O₃ / Gew. % | 3,2 | 3,2 | 3,2 |
| Cs / Gew.% | 0,4 | 0,2 | 0,1 |
| P / Gew.% | 0,2 | 0,2 | 0,2 |
| TiO₂ / Gew.% | Rest zu 100 % | Rest zu 100 % | Rest zu 100 % |
| BET TiO₂/(m²/g) | 20 | 20 | 30 |
| Anteil AM/ Gew.% | 8,0 | 7,5 | 7,5 |

### Beispiel 2: (Erfindungsgemäßes Beispiel):

Ein 4 Lagen-Katalysatorsystem mit nachfolgender Zusammensetzung und Lagenlänge wurde in einen salzbadgekühlten Rohrreaktor mit 25 mm inneren Durchmesser gefüllt. Im Reaktionsrohr befand sich zentrisch angeordnet eine 3 mm Thermohülse mit eingebautem Zugelement zur Temperaturmessung. Durch das Rohr wurden stündlich von oben nach unten 4 Nm³ Luft mit einer Beladung von 30 - 100 g o-Xylol / Nm³ Luft (Reinheit o-Xylol > 99 %) an und bei einem Gesamtdruck von ca. 1450 mbar geleitet.

Bei einer Beladung von 60 - 65 g o-Xylol / Nm³ Luft wurde bei Salzbadtemperaturen zwischen 365 und 375 °C der in Beispiel 1 betrachtete Hotspot nunmehr in der Lage 2 bei einer Position von 75 - 85 cm gemessen (vom Beginn der Schüttung in Richtung Reaktorausgang).

| Zusammensetzung | Lage 1 | Lage 2 | Lage 3 | Lage 4 |
|---|---|---|---|---|
| | Länge: 50 cm | Länge: 100 cm | Länge: 60 cm | Länge: 80 cm |
| V₂O₅ / Gew. % | 8,0 | 7,5 | 7,5 | 7,5 |
| Sb₂O₃ / Gew.% | 3,2 | 3,2 | 3,2 | 3,2 |
| Cs / Gew.% | 0,4 | 0,4 | 0,2 | 0,1 |
| P / Gew.% | 0,2 | 0,2 | 0,2 | 0,2 |
| TiO₂ / Gew.% | Rest zu 100 % | Rest zu 100 % | Rest zu 100 % | Rest zu 100 % |
| BET TiO₂ / (m²/g) | 20 | 20 | 20 | 30 |
| Anteil AM / Gew.% | 10 | 8 | 7,5 | 7,5 |

Die Lage des betrachteten Hotspots im erfindungsgemäßen Beispiel 2 ist damit signifikant näher in Richtung Reaktoreingang als im Vergleichsbeispiel 1.

Daraus können die folgenden Vorteile für den erfindungsgemäßen Katalysator abgeleitet werden, die nicht nur für das spezifische Beispiel, sondern allgemein für die vorliegende Erfindung gelten:
- Längere Lebensdauer, da der Hotspot bei Reaktionsbeginn und demzufolge auch bei fortschreitender Desaktivierung näher am Reaktoreingang liegt, insbesondere länger in der 2. Lage, (vormals 1. Lage) verbleibt.
- Geringerer Gehalt an Phthalid im Reaktionsgas, das den Reaktor verlässt, da die Reaktion weiter nach vorne verlagert ist.
- der (Neben-)Hotspot in der 3. Lage ist geringer als beim Vergleichsbeispiel in der äquivalenten 2. Lage, da mehr o-Xylol in den beiden vorhergehenden Lagen 1 und 2 umgesetzt wird als im Vergleichsbeispiel in Lage 1.

Bei einer Wiederholung von Beispiel 2, wobei der eingesetzte Katalysator bis auf die Abwesenheit von Phosphor in der ersten Lage identisch war, konnten die Salzbadtemperaturen bei gleicher Beladung etwas verringert werden und der Hotspot lag noch etwas näher zur Gaseintrittsseite (Position: ca. 70 cm).

Im folgenden wird der Einfluss einer vorgeschalteten Katalysatorlage beschrieben, in der der Cs-Gehalt zur Erhöhung der Katalysatoraktivität verringert wurde.

### Beispiel 3: (Vergleichsbeispiel):

Es wurde ein 3 Lagen-Katalysatorsystem mit nachfolgender Zusammensetzung und Lagenlänge in einen salzbadgekühlten Rohrreaktor mit 25 mm inneren Durchmesser gefüllt. Im Reaktionsrohr befand sich zentrisch angeordnet eine 3 mm Thermohülse mit eingebautem Zugelement zur Temperaturmessung. Durch das Rohr wurden stündlich von oben nach unten 4 Nm³ Luft mit einer Beladung von 30 - 100 g o-Xylol / Nm³ Luft (Reinheit o-Xylol > 99 %) an und bei einem Gesamtdruck von 1450 mbar geleitet. Bei einer Beladung von 60 - 65 g o-Xylol / Nm³ Luft wurde bei Salzbadtemperaturen zwischen 358 und 362 °C der Hotspot in der Lage 1 bei einer Position von 90 cm gemessen vom Beginn der Schüttung in Richtung Reaktorausgang.

| Zusammensetzung | Lage 1 | Lage 2 | Lage 3 |
|---|---|---|---|
| | Länge: 150 cm | Länge: 60 cm | Länge: 80 cm |
| V₂O₅ / Gew. % | 7,5 | 7,5 | 7,5 |
| Sb₂O₃ / Gew. % | 3,2 | 3,2 | 3,2 |
| Cs / Gew.% | 0,4 | 0,2 | 0 |
| P / Gew.% | 0 | 0,2 | 0,2 |
| TiO₂ / Gew. % | Rest zu 100 % | Rest zu 100 % | Rest zu 100 % |
| BET TiO₂ / (m²/g) | 20 | 20 | 30 |
| Anteil AM / Gew. % | 8,0 | 7,5 | 7,0 |

### Beispiel 4: (Erfindungsgemäßes Beispiel):

Ein 4 Lagen-Katalysatorsystem mit nachfolgender Zusammensetzung und Lagenlänge wurde in einen salzbadgekühlten Rohrreaktor mit 25 mm inneren Durchmesser gefüllt. Im Reaktionsrohr befand sich zentrisch angeordnet eine 3 mm Thermohülse mit eingebautem Zugelement zur Temperaturmessung. Durch das Rohr wurden stündlich von oben nach unten 4 Nm³ Luft mit einer Beladung von 30 - 100 g o-Xylol / Nm³ Luft (Reinheit o-Xylol > 99 %) an und bei einem Gesamtdruck von ca. 1450 mbar geleitet.

Bei einer Beladung von 60 - 65 g o-Xylol / Nm³ Luft wurde bei Salzbadtemperaturen zwischen 352 und 356 °C der hotspot in der Lage 1 bei einer Position von 80 cm gemessen vom Beginn der Schüttung in Richtung Reaktorausgang.

| Zusammensetzung | Lage 1 | Lage 2 | Lage 3 | Lage 4 |
|---|---|---|---|---|
| | Länge: 45 cm | Länge: 105 cm | Länge: 60 cm | Länge: 80 cm |
| V₂O₅ / Gew. % | 7,5 | 7,5 | 7,5 | 7,5 |
| Sb₂O₃ / Gew.% | 3,2 | 3,2 | 3,2 | 3,2 |
| Cs / Gew.% | 0,35 | 0,4 | 0,2 | 0 |
| P / Gew.% | 0 | 0 | 0,2 | 0,2 |
| TiO₂ / Gew.% | Rest zu 100 % | Rest zu 100 % | Rest zu 100 % | Rest zu 100 % |
| BET TiO₂ / (m²/g) | 20 | 20 | 20 | 30 |
| Anteil AM / Gew.% | 8 | 8 | 7,5 | 7,0 |

Die Hotspot-Lage im erfindungsgemäßen Beispiel 4 ist damit um ca. 10 cm näher in Richtung Reaktoreingang als im Vergleichsbeispiel 3.

## Patentansprüche

1. Verfahren zur Herstellung von Phthalsäureanhydrid durch Gasphasenoxidation von o-Xylol und/oder Naphthalin unter Verwendung eines Katalysators enthaltend mindestens eine erste, zur Gaseintrittsseite hin gelegene Katalysatorlage, eine zweite, näher zur Gasaustrittsseite hin gelegene Katalysatorlage und eine dritte, noch näher zur oder an der Gasaustrittsseite hin gelegene Katalysatorlage, wobei die Katalysatorlagen jeweils eine Aktivmasse enthaltend TiO₂ aufweisen, **dadurch gekennzeichnet, dass** die Katalysatoraktivität der ersten Katalysatorlage höher ist als die Katalysatoraktivität der zweiten Katalysatorlage, das Reaktionsgas bei Temperaturen von 320 bis 420 °C über den Katalysator geleitet wird und unter der Katalysatoraktivität der jeweiligen Katalysatorlage die Fähigkeit verstanden wird, innerhalb eines definierten Volumens bei vorgegebenen jeweils gleichen Reaktionsbedingungen o-Xylol und/oder Naphthalin umzusetzen.

2. Verfahren nach Anspruch 1, **dadurch gekennzeichnet, dass** die Katalysatoraktivität von der zweiten zur dritten Katalysatorlage ansteigt.

3. Verfahren nach einem der vorstehenden Ansprüche, **dadurch gekennzeichnet, dass** die Katalysatoraktivität von der dritten zur vierten und gegebenenfalls weiter zur fünften Katalysatorlage ansteigt.

4. Verfahren nach einem der vorstehenden Ansprüche, **dadurch gekennzeichnet, dass** insgesamt vier oder fünf Katalysatorlagen vorhanden sind.

5. Verfahren nach einem der vorstehenden Ansprüche, **dadurch gekennzeichnet, dass** die Länge der ersten Katalysatorlage etwa 5 bis 30 % der Gesamtlänge des gesamten Katalysatorbettes beträgt.

6. Verfahren nach einem der vorstehenden Ansprüche, **dadurch gekennzeichnet, dass** die erste Katalysatorlage
a. einen höheren Aktivmassegehalt und/oder
b. einen höheren Vanadiumgehalt und/oder
c. ein Ti0₂ mit einer höheren BET-Oberfläche und/oder
d. einen geringeren Sb-Gehalt und/oder
e. einen geringeren Cs-Gehalt und/oder
f. einen höheren Gehalt an die Aktivität steigernden Promotoren und/oder
g. eine höhere Schüttdichte und/oder
h. einen geringeren Gehalt an die Aktivität dämpfenden Promotoren
aufweist als die zweite Katalysatorlage.

7. Verfahren nach einem der vorstehenden Ansprüche, **dadurch gekennzeichnet, dass** die erste Katalysatorlage einen höheren Gehalt an die Aktivität steigernden Promotoren enthält.

8. Verfahren nach einem der vorstehenden Ansprüche, **dadurch gekennzeichnet, dass** die erste Lage eine höhere Schüttdichte aufweist als die zweite Katalysatorlage, durch Verwendung einer unterschiedlichen Geometrie der Formkörper.

9. Verfahren nach einem der vorstehenden Ansprüche, **dadurch gekennzeichnet, dass** es sich bei den einzelnen Katalysatorlagen um Schalenkatalysatoren handelt, bei denen eine Aktivmasse auf einen inerten Träger aufgebracht ist.

10. Verfahren nach einem der vorstehenden Ansprüche, **dadurch gekennzeichnet, dass** die einzelnen Katalysatorlagen als Aktivmasse zumindest enthalten:
| Zusammensetzung | Bereiche |
|---|---|
| V₂Oₛ / Gew.% | 1 - 25 |
| Sb₂0₃ / Gew.% | 0 - 4 |
| Cs / Gew.% | 0 - 1 |
| P / Gew.% | 0 - 2 |
wobei der Rest der Aktivmasse zu mindestens 90 Gew.-%, und wobei die BET-Oberfläche des verwendeten TiO₂ zwischen etwa 10 und 50 m²/mg liegt und der Anteil der Aktivmasse an dem Gesamtgewicht des Katalysators bei etwa 4 bis 20 Gew.-% liegt.

11. Verfahren nach einem der vorstehenden Ansprüche, **dadurch gekennzeichnet, dass** der Katalysator der ersten Katalysatorlage einen Aktivmassegehalt zwischen etwa 6 und 20 Gew.-% aufweist und die Aktivmasse zwischen 5 bis 16 Gew.-% V₂0₅, 0 bis 5 Gew.- Sb₂0₃, 0, 2 bis 0,75 Gew.-% Cs, 0 bis 3 Gew.-% Nb₂0₅, 0 bis 1 Gew.-% P und den Rest an Ti0₂ enthält.

12. Verfahren nach einem der vorstehenden Ansprüche, **dadurch gekennzeichnet, dass** die zweite Katalysatorlage einen Aktivmassegehalt zwischen etwa 6 und 12 Gew.-% aufweist, wobei die Aktivmasse vorzugsweise zwischen 5 bis 15 Gew.-% V₂0₅, 0 bis 5 Gew.-% Sb₂0₃, 0,2 bis 0,75 Gew.-% Cs, 0 bis 2 Gew.-% Nb₂0₅, 0 bis 1 Gew.-% P und den Rest an Ti0₂ enthält.

13. Verfahren nach einem der vorstehenden Ansprüche, **dadurch gekennzeichnet, dass** die dritte KatalysatorLage einen Aktivmassegehalt von etwa 5 bis 11 Gew.-% aufweist, wobei die Aktivmasse 5 bis 15 Gew.-% V₂0₅, 0 bis 4 Gew.-% Sb₂0₃, 0,05 bis 0,5 Gew.-% Cs, 0 bis 2 Gew.-% Nb₂0₅, 0 bis 1 Gew.-% P und den Rest an Ti0₂ enthält.

14. Verfahren nach einem der vorstehenden Ansprüche, **dadurch gekennzeichnet, dass** die vierte Katalysatorlage einen Aktivmassegehalt von 5 bis 25 Gew.-% V₂0₅, 0 bis 5 Gew.-% Sb₂0₃, 0 bis 0,2 Gew.-% Cs, 0 bis 1 Gew.-% Nb₂0₅, 0 bis 2 Gew.-% P und den Rest an Ti0₂ enthält.

15. Verfahren nach einem der vorstehenden Ansprüche, **dadurch gekennzeichnet, dass** die erste Katalysatorlage einen Aktivmassegehalt zwischen etwa 7 und 20 Gew.-% aufweist,
die zweite Katalysatorlage einen Aktivmassegehalt zwischen etwa 7 und 12 Gew.-% aufweist, wobei der Aktivmassegehalt der zweiten Katalysatorlage kleiner oder gleich dem Aktivmassegehalt der ersten Katalysatorlage ist;
die dritte Katalysatorlage einen Aktivmassegehalt im Bereich zwischen etwa 6 und 11 Gew.-% aufweist, wobei der Aktivmassegehalt der dritten Katalysatorlage kleiner oder gleich dem Aktivmassegehalt der zweiten Katalysatorlage ist, und
die vierte Katalysatorlage einen Aktivmassegehalt im Bereich zwischen 5 und 10 Gew.-% aufweist, wobei der Aktivmassegehalt der vierten Katalysatorlage kleiner oder gleich dem Aktivmassegehalt der dritten Katalysatorlage ist.

16. Verfahren nach einem der vorstehenden Ansprüche, **dadurch gekennzeichnet, dass** die BET-Oberfläche der letzten, zur Gasaustrittsseite hin gelegenen Katalysatorlage höher ist als die BET-Oberfläche der vorausgehenden (gasstromauf gelegenen) Katalysatorlagen.

17. Verfahren nach einem der vorstehenden Ansprüche, **dadurch gekennzeichnet, dass** mindestens etwa 40% des gesamten Porenvolumens des eingesetzten Ti0₂ durch Poren mit einem Radius zwischen 60, und 400 nm gebildet werden.

18. Verfahren nach einem der vorstehenden Ansprüche, **dadurch gekennzeichnet, dass** bis zu 75%, des gesamten Porenvolumens des eingesetzten Ti0₂ durch Poren mit einem Radius zwischen 60 und 400 nm gebildet werden.

19. Verfahren nach einem der vorstehenden Ansprüche, **dadurch gekennzeichnet, dass** die katalytisch aktive Masse im Fließ- bzw. Wirbelbett aufgebracht wird.

20. Verfahren nach einem der vorstehenden Ansprüche, **dadurch gekennzeichnet, dass** in mindestens einer Katalysatorlage mindestens 0,05 Gew.-% der katalytisch aktiven Masse aus mindestens einem Alkalimetall, berechnet als Alkalimetall(e), gebildet wird.

21. Verfahren nach einem der vorstehenden Ansprüche, **dadurch gekennzeichnet, dass** als Kleber für die katalytisch aktive Masse ein organisches Polymer oder Copolymer verwendet wird.

22. Verfahren nach einem der vorstehenden Ansprüche, **dadurch gekennzeichnet, dass** der Katalysator mindestens 24 Stunden bei > 390°C in einem O₂-haltigen Gas calciniert bzw. konditioniert wird.

23. Verfahren nach einem der vorstehenden Ansprüche, **dadurch gekennzeichnet, dass** in mindestens einer Katalysatorlage Niob in einer Menge von 0,1 bis 2 Gew.-% der katalytisch aktiven Masse vorhanden ist.

24. Verfahren nach einem der vorstehenden Ansprüche, **dadurch gekennzeichnet, dass** nur eine TiO₂-Quelle verwendet wird, wobei das gesamte eingesetzte Ti0₂ die in einem oder mehreren der vorstehenden Ansprüche definierte BET-Oberfläche bzw. Porenradienverteilung aufweist.

25. Verfahren nach einem der vorstehenden Ansprüche, **dadurch gekennzeichnet, dass** mindestens in der letzten Katalysatorlage Phosphor in der aktiven Masse enthalten ist.

26. Verfahren nach einem der vorstehenden Ansprüche, **dadurch gekennzeichnet, dass** die erste Katalysatorlage eine um mindestens 5 % höhere Aktivitäten aufweist als die nachfolgende zweite Katalysatorlage.

## Claims

1. Process for the preparation of phthalic anhydride by gas-phase oxidation of o-xylene and/or naphthalene using a catalyst comprising at least a first catalyst layer situated towards the gas inflow side, a second catalyst layer situated closer towards the gas outflow side, and a third catalyst layer situated even closer towards, or at, the gas outflow side, wherein the catalyst layers each have an active mass that contains TiO₂, **characterized in that** the catalyst activity of the first catalyst layer is higher than the catalyst activity of the second catalyst layer, the reaction gas is passed over the catalyst at temperatures from 320 to 420°C and the catalyst activity of each catalyst layer is understood as meaning the ability to react o-xylene and/or naphthalene within a defined volume under the specified, in each case identical, reaction conditions.

2. Process according to Claim 1, **characterized in that** the catalyst activity increases from the second to the third catalyst layer.

3. Process according to either of the preceding claims, **characterized in that** the catalyst activity increases from the third to the fourth, and if present on to the fifth, catalyst layer.

4. Process according to any of the preceding claims, **characterized in that** a total of four or five catalyst layers are present.

5. Process according to any of the preceding claims, **characterized in that** the length of the first catalyst layer is approximately 5 to 30% of the total length of the entire catalyst bed.

6. Process according to any of the preceding claims, **characterized in that** the first catalyst layer has
a. a higher active mass content and/or
b. a higher vanadium content and/or
c. a TiO₂ with a higher BET surface area and/or
d. a lower Sb content and/or
e. a lower Cs content and/or
f. a higher content of activity-boosting promoters and/or
g. a higher bulk density and/or
h. a lower content of activity-suppressing promoters
than the second catalyst layer.

7. Process according to any of the preceding claims, **characterized in that** the first catalyst layer contains a higher content of activity-boosting promoters.

8. Process according to any of the preceding claims, **characterized in that** the first layer has a higher bulk density than the second catalyst layer due to use of a different geometry for the moulded body.

9. Process according to any of the preceding claims, **characterized in that** the individual catalyst layers are shell catalysts in which an active mass is applied to an inert support.

10. Process according to any of the preceding claims, **characterized in that** the individual catalyst layers contain, as active mass, at least the following:
| Composition | Ranges |
|---|---|
| V₂O₅ / % by weight | 1 - 25 |
| Sb₂O₃ / % by weight | 0 - 4 |
| Cs / % by weight | 0 - 1 |
| P / % by weight | 0 - 2 |
wherein the remainder of the active mass not less than 90% by weight and wherein the BET surface area of the TiO₂ used is between approximately 10 and 50 m²/g and the proportion of the active mass based on the total catalyst weight is approximately 4 to 20% by weight.

11. Process according to any of the preceding claims, **characterized in that** the catalyst in the first catalyst layer has an active mass content of between approximately 6 and 20% by weight and the active mass contains between 5 to 16% by weight of V₂O₃, 0 to 5% by weight of Sb₂O₃, 0.2 to 0.75% by weight of Cs, 0 to 3% by weight of Nb₂O₅, 0 to 1% by weight of P, remainder TiO₂.

12. Process according to any of the preceding claims, **characterized in that** the second catalyst layer has an active mass content of between approximately 6 and 12% by weight, wherein the active mass preferably contains between 5 to 15% by weight of V₂O₅, 0 to 5% by weight of Sb₂O₃, 0.2 to 0.75% by weight of Cs, 0 to 2% by weight of Nb₂O₅, 0 to 1% by weight of P, remainder TiO₂.

13. Process according to any of the preceding claims, **characterized in that** the third catalyst layer has an active mass content from approximately 5 to 11% by weight, wherein the active mass contains 5 to 15% by weight of V₂O₅, 0 to 4% by weight of Sb₂O₃, 0.05 to 0.5% by weight of Cs, 0 to 2% by weight of Nb₂O₅, 0 to 1% by weight of P, remainder TiO₂.

14. Process according to any of the preceding claims, **characterized in that** the fourth catalyst layer has an active mass content from 5 to 25% by weight of V₂O₅, 0 to 5% by weight of Sb₂O₃, 0 to 0.2% by weight of Cs, 0 to 1% by weight of Nb₂O₅, 0 to 2% by weight of P, remainder TiO₂.

15. Process according to any of the preceding claims, **characterized in that** the first catalyst layer has an active mass content of between approximately 7 and 20% by weight,
the second catalyst layer has an active mass content of between approximately 7 and 12% by weight, wherein the active mass content of the second catalyst layer is less than or equal to the active mass content of the first catalyst layer;
the third catalyst layer has an active mass content in the range between approximately 6 and 11% by weight, wherein the active mass content of the third catalyst layer is less than or equal to the active mass content of the second catalyst layer, and
the fourth catalyst layer has an active mass content in the range between 5 and 10% by weight, wherein the active mass content of the fourth catalyst layer is less than or equal to the active mass content of the third catalyst layer.

16. Process according to any of the preceding claims, **characterized in that** the BET surface area of the final catalyst layer situated towards the gas outflow side is greater than the BET surface area of the preceding (upstream) catalyst layers.

17. Process according to any of the preceding claims, **characterized in that** not less than 40% of the total pore volume of the TiO₂ used is made up of pores with a radius of between 60 and 400 nm.

18. Process according to any of the preceding claims, **characterized in that** up to 75% of the total pore volume of the TiO₂ used is made up of pores with a radius of between 60 and 400 nm.

19. Process according to any of the preceding claims, **characterized in that** the catalytically active mass is applied in a fluid/fluidized bed.

20. Process according to any of the preceding claims, **characterized in that** in at least one catalyst layer not less than 0.05% by weight of the catalytically active mass is made up of at least one alkali metal, calculated as alkali metal(s).

21. Process according to any of the preceding claims, **characterized in that** the adhesive used for the catalytically active mass is an organic polymer or copolymer.

22. Process according to any of the preceding claims, **characterized in that** the catalyst is calcined/conditioned in an O₂-containing gas for at least 24 hours at > 390°C.

23. Process according to any of the preceding claims, **characterized in that**, in at least one catalyst layer, niobium is present in an amount from 0.1 to 2% by weight of the catalytically active mass.

24. Process according to any of the preceding claims, **characterized in that** only one TiO₂ source is used, wherein all of the TiO₂ used has the BET surface area and pore radius distribution defined in one or more of the preceding claims.

25. Process according to any of the preceding claims, **characterized in that** at least in the final catalyst layer phosphorus is present in the active mass.

26. Process according to any of the preceding claims, **characterized in that** the first catalyst layer has an activity at least 5% higher than the subsequent second catalyst layer.

## Revendications

1. Procédé de fabrication d'anhydride de l'acide phtalique par oxydation en phase gazeuse d'o-xylène et/ou de naphtaline en utilisant un catalyseur contenant au moins une première couche de catalyseur disposée vers le côté de l'entrée de gaz, une deuxième couche de catalyseur disposée plus près du côté de la sortie de gaz, et une troisième couche de catalyseur disposée encore plus près du ou sur le côté de la sortie de gaz, les couches de catalyseur comprenant chacune une masse active contenant du TiO₂, **caractérisé en ce que** l'activité catalytique de la première couche de catalyseur est plus élevée que l'activité catalytique de la deuxième couche de catalyseur, le gaz réactionnel est conduit sur le catalyseur à des températures allant de 320 à 420 °C, et l'activité catalytique de chaque couche de catalyseur se rapporte à l'aptitude à transformer l'o-xylène et/ou la naphtaline dans un volume défini et dans des conditions de réaction prédéterminées, à chaque fois identiques.

2. Procédé selon la revendication 1, **caractérisé en ce que** l'activité catalytique augmente depuis la deuxième vers la troisième couche de catalyseur.

3. Procédé selon l'une quelconque des revendications précédentes, **caractérisé en ce que** l'activité catalytique augmente depuis la troisième vers la quatrième et éventuellement encore vers la cinquième couche de catalyseur.

4. Procédé selon l'une quelconque des revendications précédentes, **caractérisé en ce que** quatre ou cinq couches de catalyseur sont présentes au total.

5. Procédé selon l'une quelconque des revendications précédentes, **caractérisé en ce que** la longueur de la première couche de catalyseur est d'environ 5 à 30 % de la longueur totale du lit catalytique entier.

6. Procédé selon l'une quelconque des revendications précédentes, **caractérisé en ce que** la première couche de catalyseur présente :
a. une teneur en masse active plus élevée et/ou
b. une teneur en vanadium plus élevée et/ou
c. un TiO₂ ayant une surface BET plus élevée et/ou
d. une teneur en Sb plus faible et/ou
e. une teneur en Cs plus faible et/ou
f. une teneur plus élevée en promoteurs augmentant l'activité et/ou
g. une densité apparente plus élevée et/ou
h. une teneur plus faible en promoteurs atténuant l'activité
que la deuxième couche de catalyseur.

7. Procédé selon l'une quelconque des revendications précédentes, **caractérisé en ce que** la première couche de catalyseur présente une teneur plus élevée en promoteurs augmentant l'activité.

8. Procédé selon l'une quelconque des revendications précédentes, **caractérisé en ce que** la première couche présente une densité apparente plus élevée que la deuxième couche de catalyseur, en raison de l'utilisation d'une géométrie différente des corps moulés.

9. Procédé selon l'une quelconque des revendications précédentes, **caractérisé en ce que** les couches de catalyseur individuelles consistent en des catalyseurs à enveloppe, dans lesquels une masse active est appliquée sur un support inerte.

10. Procédé selon l'une quelconque des revendications précédentes, **caractérisé en ce que** les couches de catalyseur individuelles contiennent en tant que masse active au moins :
| Composition | Plage |
|---|---|
| V₂O₅ / % en poids | 1 à 25 |
| Sb₂O₃ / % en poids | 0 à 4 |
| Cs / % en poids | 0 à 1 |
| P / % en poids | 0 à 2 |
le reste de la masse active d'au moins 90 % en poids, et la surface BET du TiO₂ utilisé étant comprise entre environ 10 et 50 m²/mg, et la proportion de la masse active par rapport au poids total du catalyseur étant d'environ 4 à 20 % en poids.

11. Procédé selon l'une quelconque des revendications précédentes, **caractérisé en ce que** le catalyseur de la première couche de catalyseur présente une teneur en masse active comprise entre environ 6 et 20 % en poids, et la masse active contient entre 5 et 16 % en poids de V₂O₅, 0 à 5 % en poids de Sb₂O₃, 0,2 à 0,75 % en poids de Cs, 0 à 3 % en poids de Nb₂O₅, 0 à 1 % en poids de P, le reste étant du TiO₂.

12. Procédé selon l'une quelconque des revendications précédentes, **caractérisé en ce que** la deuxième couche de catalyseur présente une teneur en masse active comprise entre environ 6 et 12 % en poids, la masse active contenant de préférence de 5 à 15 % en poids de V₂O₅, 0 à 5 % en poids de Sb₂O₃, 0,2 à 0,75 % en poids de Cs, 0 à 2 % en poids de Nb₂O₅, 0 à 1 % en poids de P, le reste étant du TiO₂.

13. Procédé selon l'une quelconque des revendications précédentes, **caractérisé en ce que** la troisième couche de catalyseur présente une teneur en masse active d'environ 5 à 11 % en poids, la masse active contenant de 5 à 15 % en poids de V₂O₃, 0 à 4 % en poids de Sb₂O₃, 0,05 à 0,5 % en poids de Cs, 0 à 2 % en poids de Nb₂O₅, 0 à 1 % en poids de P, le reste étant du TiO₂.

14. Procédé selon l'une quelconque des revendications précédentes, **caractérisé en ce que** la quatrième couche de catalyseur présente une teneur en masse active de 5 à 25 % en poids de V₂O₃, 0 à 5 % en poids de Sb₂O₃, 0 à 0,2 % en poids de Cs, 0 à 1 % en poids de Nb₂O₅, 0 à 2 % en poids de P, le reste étant du TiO₂.

15. Procédé selon l'une quelconque des revendications précédentes, **caractérisé en ce que** la première couche de catalyseur présente une teneur en masse active comprise entre environ 7 et 20 % en poids,
la deuxième couche de catalyseur présente une teneur en masse active comprise entre environ 7 et 12 % en poids, la teneur en masse active de la deuxième couche de catalyseur étant inférieure ou égale à la teneur en masse active de la première couche de catalyseur ;
la troisième couche de catalyseur présente une teneur en masse active dans la plage comprise entre environ 6 et 11 % en poids, la teneur en masse active de la troisième couche de catalyseur étant inférieure ou égale à la teneur en masse active de la deuxième couche de catalyseur, et
la quatrième couche de catalyseur présente une teneur en masse active dans la plage comprise entre 5 et 10 % en poids, la teneur en masse active de la quatrième couche de catalyseur étant inférieure ou égale à la teneur en masse active de la troisième couche de catalyseur.

16. Procédé selon l'une quelconque des revendications précédentes, **caractérisé en ce que** la surface BET de la dernière couche de catalyseur disposée vers le côté de la sortie de gaz est plus élevée que la surface BET des couches de catalyseur précédentes (agencées en amont du gaz) .

17. Procédé selon l'une quelconque des revendications précédentes, **caractérisé en ce qu'**au moins environ 40 % du volume poreux total du TiO₂ utilisé est formé par des pores ayant un rayon compris entre 60 et 400 nm.

18. Procédé selon l'une quelconque des revendications précédentes, **caractérisé en ce que** jusqu'à 75 % du volume poreux total du TiO₂ utilisé est formé par des pores ayant un rayon compris entre 60 et 400 nm.

19. Procédé selon l'une quelconque des revendications précédentes, **caractérisé en ce que** la masse catalytiquement active est agencée dans un lit fluidisé.

20. Procédé selon l'une quelconque des revendications précédentes, **caractérisé en ce qu'**au moins 0,05 % en poids de la masse catalytiquement active est formée par au moins un métal alcalin, calculé en tant que métal ou métaux alcalins, dans au moins une couche de catalyseur.

21. Procédé selon l'une quelconque des revendications précédentes, **caractérisé en ce qu'**un polymère ou copolymère organique est utilisé en tant qu'adhésif pour la masse catalytiquement active.

22. Procédé selon l'une quelconque des revendications précédentes, **caractérisé en ce que** le catalyseur est calciné ou conditionné pendant au moins 24 heures à > 390 °C dans un gaz contenant de l'O₂.

23. Procédé selon l'une quelconque des revendications précédentes, **caractérisé en ce que** du niobium est présent en une quantité de 0,1 à 2 % en poids de la masse catalytiquement active dans au moins une couche de catalyseur.

24. Procédé selon l'une quelconque des revendications précédentes, **caractérisé en ce qu'**uniquement une source de TiO₂ est utilisée, l'ensemble du TiO₂ utilisé présentant la surface BET et la distribution de rayons de pores définies dans une ou plusieurs des revendications précédentes.

25. Procédé selon l'une quelconque des revendications précédentes, **caractérisé en ce que** du phosphore est contenu dans la masse active au moins dans la dernière couche de catalyseur.

26. Procédé selon l'une quelconque des revendications précédentes, **caractérisé en ce que** la première couche de catalyseur présente une activité au moins 5 % plus élevée que la deuxième couche de catalyseur suivante.
